# EUROPEAN PATENT APPLICATION

(11) **EP 1 780 412 A2**
(43) Date of publication of application: **02.05.2007**
(21) Application number: 06255358.1
(22) Date of filing: 18.10.2006
(51) Int. Cl.: F04B 19/00

(54) **Devices with a passageway for electroosmotic flow and method of making same**

(30) Priority: 28.10.2005 GB 0522063
(71) Applicant: The University of Hull, Hull HU6 7RX (GB)
(72) Inventor: Watts, Paul, Victoria Dock Hull HU9 1PW (GB); Wiles, Charlotte, Hedon Hull HU12 8PH (GB)
(74) Representative: Hartley, Andrew Philip

(57) **Abstract**

A device has a passageway (14) for electroosmotic flow therealong. The passageway (14) is bordered by an internal surface (12). A porous plug (16,18) is located in the passageway (14) so that there are no gaps between the porous plug (16, 18) and the internal surface (12). The plug (16,18) is made by curing a paste comprising a filler, such as glass powder, and a silicate solution. The passageway preferably has a maximum cross-sectional dimension of greater than 500µm, a minimum cross-sectional dimension of greater than 500µm or a cross-sectional area of greater than 0.2mm^{2.}

## Description

The invention relates to a method of making a device for electroosmotic flow therein, a device with a passageway for electroosmotic flow therein, and a method of generating electroosmotic flow. Additionally, the invention relates to a method of preparing a 1,3-dithiane or a 1,2-dithiolane.

Devices with passageways for electroosmotic flow therein are well known in the art. For example a first type of known device is described in US 6,344,120, in a review article by Haswell S. J. in the Analyst, January 1997, Volume 122 (1R-10R), and in a review article by Fletcher et al in Tetrahedron, 58 (2002), 4735-4757. In this first type of known device, the device comprises a first member with a groove and a second member with a surface. The two members are attached to one another so that the surface closes the groove to form a passageway. A porous silica plug is located at a convenient point within the passageway. Methods of making such a device are described in the three documents referred to above. In particular, the porous silica plug, otherwise known as a frit, is prepared from a mixture of formamide and potassium silicate solutions. Before the two members are attached to one another, a drop of the solution is placed into the groove in the first member at a desired position. The solution is then heat cured, in the groove, to form a microporous silica structure.

The second member is then bonded to the first member so as to seal the groove with the microporous silica structure forming a plug in the passageway. The method of forming the microporous silica structure is described in detail in article by Christensen et al in Anal.Commun., 1998, Volume 35, 341-343, and also in US 6,344,120.

A second type of device with a passageway for electroosmotic flow therein is described in the article by Christensen et al referred to above. In this second type of device, a microporous silica plug is formed in the passageway of capillary tube. The porous silica plug is formed in the same way as described above.

A problem with these known devices is that some shrinkage of the mixture of formamide and potassium silicate solution occurs during heat curing. This leads to small gaps between the porous silica plugs and the surfaces bordering the passageways in which they are located. This is disadvantageous as solutions passing along the passageways can by-pass the porous silica plugs by passing through these gaps.

In the known devices described above, electroosmotic flow of liquid can be induced along the passageway, and through the pores of the porous silica plug, by applying an electric voltage across the length of the passageway. However, it has been found that in the known devices described above, electroosmotic flow will only occur in passageways which are relatively narrow - that is to say in passageways which have a maximum cross-sectional dimension of 500 µm or less. The maximum cross-sectional dimension is the longest straight line which extends across the passageway in a cross-sectional plane. This limitation in the maximum cross-sectional dimension is disadvantageous as it restricts the flow rates that can be achieved by electroosmotic flow in the passageways of these devices.

According to a first aspect of the invention, there is provided a method of making a device for electroosmotic flow therein, comprising, preparing a device with a passageway therein and with a porous plug in the passageway, the porous plug being formed by curing a paste comprising filler particles and a silicate solution, the device having an internal surface which borders the passageway and which is contacted by the plug all around the passageway without any gaps therebetween.

According to a second aspect of the invention, there is provided a device made by a method in accordance with the first aspect of the invention.

In accordance with a third aspect of the invention, there is provided a device with a passageway for electroosmotic flow therein, the device having an internal surface which borders the passageway, a porous plug within the passageway, the plug contacting the surface all around the passageway without any gaps therebetween.

According to a fourth aspect of the invention, there is provided a device according to the third aspect of the invention, made by a method in accordance with the first aspect of the invention.

In accordance with a fifth aspect of the invention, there is provided a method of generating electroosmotic flow comprising providing a device in accordance with any one of the second, third or fourth aspects of the invention, filling the passageway and the pores of the porous plug with a liquid, and applying an electric voltage across the length of the passageway to cause electroosmotic flow therealong.

In accordance with a sixth aspect of the invention, there is provided a method of preparing a 1,3-dithiane or a 1,2-dithiolane, comprising mixing an aldehyde or a ketone with a dithiol and passing the mixture through a supported acid catalyst so as to produce a 1,3-dithiane or a 1,2-dithiolane.

The passageways of the current invention (and also the passageways of the known devices described above) are not necessarily circular in cross-section. In general, the passageways may have any cross-sectional shape including, for example, circular, D-shapes and rectangular. The term maximum cross-sectional dimension is defined above. As used herein, the minimum cross-sectional dimension refers to the shortest straight line which extends, in a cross-section, fully across the passageway and through the mid-point of the passageway. For example, if a passageway has a circular cross-section, the minimum cross-sectional dimension will be a diameter. If the passage has a rectangular cross-section, then the minimum cross-sectional dimension will be the line which passes through the centre of the passageway, between and perpendicular to the closest sides. The passageways of the current invention preferably have a regular cross-sectional shape.

As mentioned above, known devices with passageways for electroosmotic flow have passageways with a maximum cross-sectional dimension (as defined above) of 500 µm or less. In the known devices, electroosmotic flow is unachievable if the maximum cross-sectional dimension is greater than 500µm. However, in the current invention, the maximum cross-sectional dimension of the passageway is preferably greater than 500µm. More preferably, the maximum cross-sectional dimension is greater than 600µm, or 700µm, or 800µm, or 900µm, or 1000µm, or 1200µm, or 1400µm, or 1600µm, or 1800µm, or 2000µm (the greater the dimension, the more preferred). These maximum cross-sectional dimensions preferably apply to the whole length of the passageway. It has been found that electroosmotic flow surprisingly can take place in such passageways.

Alternatively, the minimum cross-sectional dimension of the passageway of the current invention, as defined above, is preferably greater than 500µm, and more preferably greater than 600µm , or 700µm, or 800µm, or 900µm, or 1000µm, or 1200µm, or 1400µm, or 1600µm, or 1800µm, or 2000µm (the greater the dimension, the more preferred). These minimum cross-sectional dimensions preferably apply to the whole length of the passageway. It has been found that electroosmotic flow surprisingly can take place in such passageways.

In known devices having passageways for electroosmotic flow, the cross-sectional area of the passageways does not exceed 0.2 mm². This is because electroosmotic flow does not occur in passageways having greater cross-sectional areas in devices of known type. However, the passageways of the current invention preferably have a cross-sectional area of greater than 0.2 mm², more preferably greater than 0.3mm², even more preferably greater than 0.4mm², and most preferably greater than 0.5mm². Surprisingly, it has been found that passageways in accordance with the invention having cross-sectional areas of this magnitude can still support electroosmotic flow. These cross-sectional areas preferably apply to the whole length of the passageway.

The following is a more detailed description of embodiments of the invention, byway of example only, reference being made to the appended drawings in which:
Figure 1 is a schematic view of a first device in accordance with the invention;
Figure 2 is a schematic view of a second device in accordance with the invention;
Figure 3 is a schematic view of a third device in accordance with the invention and at a stage in its manufacture prior to completion; and
Figure 4 is an enlarged cross-section through a lower glass plate of the third device.

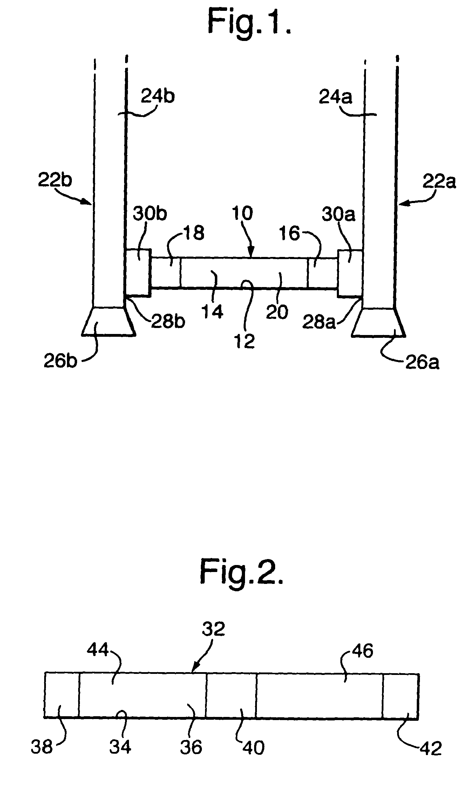

Figure 1 shows a flow reactor. The reactor includes a cylindrical tube 10 which may be made, for example, of glass, such as borosilicate glass, or of a polymer such as SU-8, PEEK or Teflon. The tube 10 has an internal surface 12 which borders a cylindrical passageway 14.

First and second porous plugs 16,18 are located within the passageway 14 at opposite ends of the passageway 14. Each plug 16,18 contacts the internal surface 12 of the tube 10 all around the passageway 14 so that there is no gap (other than those formed by the pores of the porous plug) between the porous plug 16,18 and the internal surface 12. Each one of the plugs 16,18 is formed from borosilicate glass powder held together in a solid silica matrix. In this example, the glass powder is of a type commercially available under the name VitraPOR (trade mark) and has a particle size of 5µm. However, other glass powders may be used. Suitable particle sizes may be between 1 and 20µm, preferably between 2.5 µm and 10µm.

An enclosed area 20 is located in the middle of the passageway 14 between the first and second porous plugs 16,18. This enclosed area 20 may include a supported reagent or catalyst of known type, and examples of suitable supported reagents and catalysts are given below.

The preparation of the tube 10, and in particular of the first and second porous plugs 16,18, will now be described. As a first step, the first plug 16 is formed at one end of the passageway 14. This is done as follows. Firstly, a potassium silicate solution is prepared which contains 21 % SiO₂ and 9% K₂O. The solvent is water. One volume of the potassium silicate solution is then mixed with two volumes of borosilicate glass powder having a particle size of 5 micrometres (or any other suitable particle size as described above). After thorough mixing, a paste is formed. A volume of the paste is introduced into the passageway 14 at one end thereof, and the tube is then heated at 100°C for 24 hours so as to cure the paste. During the curing process, the paste hardens into the first porous plug 16. Advantageously, the paste does not undergo shrinking and no gaps are formed between the first porous plug 16 and the internal surface 12. The tube 10 now has a first plug 16 formed at one end of the passageway 14.

If desired, a supported reagent, or a supported catalyst, is introduced into the passageway 14 with the tube 10 in a vertical position so that the supported reagent or catalyst rests on the porous plug 16. The second plug 18 is then formed at the other end of the passageway 14, in an identical manner to the formation of the first plug 16, so that the supported reagent or catalyst becomes trapped in the passageway 14 between the first and second plugs 16,18.

In the current example, the glass tube 10 has an internal diameter of 3 millimetres and a length of about 3 centimetres. However, these dimensions are not critical and any suitable dimensions may be used.

The flow device shown in Figure 1 also includes two identical supports 22a and 22b. Only one support will be described in detail and this support will be given reference numerals for its components having the suffix a. The other support (not described) will be given corresponding reference numerals with the suffix b.

The support 22a has a hollow cylindrical reservoir 24a which is open at a top end and closed at a bottom end. The reservoir 24a is held in a vertical position by a stand 26a attached to the bottom end of the reservoir 24a. The reservoir 24a is provided, towards its closed bottom end with a generally circular aperture 28a which is provided with a rubber seal 30a.

The aperture 28a and the rubber seal 30a are dimensioned and designed such that the tube 10 may be inserted through the rubber seal 30a and through the aperture 28a so that the rubber seal 30a provides a fluid tight seal between the aperture 28a and the tube 10. In this way, the passageway 14, and most immediately the first porous plug 16, is in direct fluid communication with the interior of the reservoir 24a.

The other end of the tube 10 is inserted through the rubber seal 30b and the aperture 28b of the other support 22b in a similar manner. In this way, the passageway 14, and most directly the second porous plug 18 is in direct fluid communication with the reservoir 24b.

Methods of using the flow reactor shown in Figure 1 are described below.

A second flow reactor is shown in Figure 2. The second flow reactor includes a tube 32 which may be formed of glass, such as borosilicate glass, or of a suitable polymer. The tube 32 defines an internal cylindical passageway 36 which is bordered by an internal surface 34. A first porous plug 38 is formed at one end of the passageway 36, a second porous plug 40 is formed approximately in the middle of the passageway 36, and a third porous plug 42 is formed at the other end of the passageway 36. The three porous plugs 38,40,42 are identical to the first and second plugs 16,18 of the flow reactor shown in Figure 1, and they are formed using the same procedure. Each of the first, second and third plugs 38,40,42 contacts the internal surface 34 all around the passageway 36 so that there is no gap between the plug and the passageway 36. A first enclosed area 44 is formed between the first and second plugs 38,40 and the second enclosed area 36 is formed between the second and third plugs 40,42. If desired, and as discussed in more detail below, the first enclosed area 44 may contain a supported reagent or catalyst and the second enclosed area 46 may contain a different supported reagent or catalyst.

The second flow reactor shown in Figure 2 may be mounted between the supports 22a,22b described above, in place of the tube 10 shown in Figure 1.

The tube 32 of the second flow reactor may have an internal diameter of about 3 millimetres and a length of about 6 centimetres, although these dimensions are not critical and any suitable dimensions may be used.

A method of using the second flow reactor will be described below.

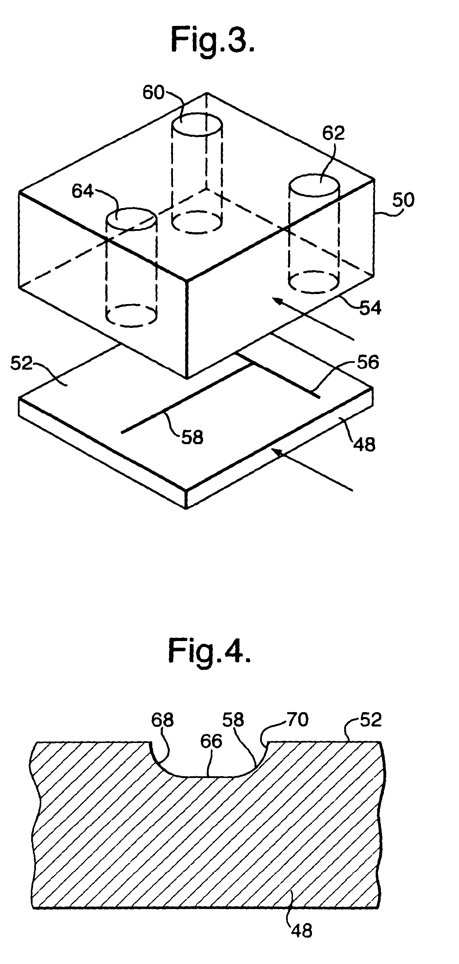

Figure 3 shows a lower glass block 48 and an upper glass block 50 which are used in the manufacture of a third flow reactor.

The lower glass block 48 has an upper surface 52 in which is formed a first groove 56 and a second groove 58 which extends from a mid-point of the first groove 56 so as to form a T formation.

The upper glass block 50 has first, second and third cylindrical holes 60,62 and 64 extending therethrough.

The upper and lower glass blocks 48,50 described above are similar to conventional blocks described in, for example, U.S. 6,344,120, the 1997 Review Article by Haswell et al referred to above, and in the 2002 Review Article by Fletcher et al referred to above. Full descriptions of how the first and second grooves 56,58 are formed are given in each of these three documents. The first and second grooves 56,58 have a generally D-shaped cross-section, as shown in Figure 4. As shown in Figure 4, which shows a cross-section through the second groove 58, each groove is bordered, at its base, by a generally planar surface 66 which is generally parallel to the upper surface 52 of the lower glass block 48. At each side of the groove 56,58 a respective curved surface 68,70 joins the generally planar surface 66 to the upper surface 52.

The D cross-sectional shape of the first and second grooves 56,58 is similar to the shape of the grooves shown in Figure 2 of the 2002 Review Article by Fletcher et al. However, in contrast to the known flow reactors described in that article, the first and second grooves 56,58 have larger dimensions. In the current example, the depth of the first and second grooves 56,58 may be 500 µm or greater. Preferably, the depth will be 1000 µm or greater. The width of the first and second grooves 56,58 is greater than their depth.

Before assembling the upper and lower glass blocks 48,50 so as to form the third flow reactor, a porous structure is formed in a suitable position in one of the first and second grooves 56,58. For example, the porous structure may be formed at the end of the second groove 58 adjacent to the first groove 56.

The porous structure is formed using the same paste mixture described above for the formation of the plugs 16,18, 38,40,42. A suitable volume of this paste is placed in the groove 58 so as to completely fill the groove and so as to be approximately flush with the upper surface 52 of the lower glass block 48. The paste mixture is then heat cured as described above so as to form a solid porous structure.

The lower surface 54 of the upper glass block 50 is then placed against the upper surface 52 of the lower glass block 48 and the glass blocks 48,50 are heat-annealed to one another. This process is well known and described, for example, in the above-mentioned review article by Fletcher et al and in U.S. 6,344,120.

After this heat-annealing process, the lower surface 54 of the upper glass block 50 closes the first and second grooves 56,58 to form, respectively, first and second interconnecting passageways (not shown). The porous structure described above in the second groove 58 now forms a plug in the second passageway. As the pasty mixture does not shrink during the curing process, this plug entirely fills the second passageway. Specifically, there is no gap between the plug and any of the surfaces surrounding it. This includes the planar surface 66 and the two curved surfaces 68,70 of the lower glass block 48, and also the lower surface 54 of the upper glass block 50.

The dimensions of the passageways formed from the first and second grooves 56,58 will generally be the same as those of the first and second grooves 56,58.

As can be seen from Figure 3, the first hole 60 now lies above one end of the first passageway, the second hole 62 now lies above the other end of the first passageway, and the third hole 64 now lies above the free end of the second passageway. The third flow reactor can be used to perform chemical reactions as for similar conventional reactors known in the art - with electroosmotic liquid flow taking place in the passageways.

It will be appreciated that the first, second and third flow reactors described above may be altered in many different ways. For example, the pasty mixture used to form the plugs need not be as described above. Whereas the pasty mixture will preferably include glass powder, and more preferably borosilicate glass powder, other suitable filler particles may be used. Suitable filler particles may be chosen both with regard to their inert nature and their surface charge. Specifically, filler particles will be inert with whichever electroosmotic liquid the flow reactor is intended for use with. Additionally, the filler particles will be chosen, preferably, so as to have a surface charge, preferably a negative surface charge, in the electroosmotic liquid with which the flow reactor is intended to be used. Other suitable silicate solutions such as sodium silicate may be used.

The passageways of the flow reactors may have any internal dimensions. However, the maximum cross-sectional dimension, as defined above, is preferably greater than 500µm, and more preferably greater than 600µm, 700µm, 800µm, 900µm, 1000µm, 1200µm, 1400µm, 1600µm, 1800µm or 2000µm (the greater dimensions being most preferred).

Alternatively, the minimum cross-sectional dimension, as defined above, is preferably greater than 500µm, and more preferably greater than 600µm, or 700µm, or 800µm, or 900µm, or 1000µm, or 1200µm, or 1400µm, or 1600µm, or 1800µm, or 2000µm (the greater diemension being most preferred).

As a further alternative, the passageways of the current invention preferably have a cross-sectional area of greater than 0.2mm², more preferably greater than 0.3 mm², even more preferably greater than 0.4 mm², and most preferably greater than 0.5 mm².

The passageways may vary in their cross-sectional dimensions and cross-sectional areas along their length. However, it is preferred that the maximum cross-sectional dimensions, or the minimum cross-sectional dimensions, or the cross-sectional areas discussed above are exceeded throughout the length of the passageways.

Flow reactors of the type shown in Figure 3, which are formed with a first member having a groove and a second member having a surface which closes the groove so as to form a passageway, can have any number of interconnecting passageways in any desired configuration. Suitable configurations are well known in the art.

In the first and second flow reactors described above the enclosed spaces contain supported reagents or catalysts - the reagents or catalysts being supported on a particulate support, such as particulate silica. Preferably, all of those areas in the passageways of the current invention which are not filled by a porous plug as described above, are provided with a particulate material. This particulate material may be a supported reagent or catalyst or mixtures thereof. Alternatively, the particulate material may simply be chemically inert. The particulate material facilitates electroosmotic flow. Preferably, the particulate material will have a surface charge in the electroosmotic liquid which will be used.

The following are examples of how the flow reactors described above may be used to perform chemical reactions.

### Synthesis of 1,3-Dithianes, and 1,2-Dithiolanes

In this reaction, a dithiol is reacted with either an aldehyde or a ketone, using a supported acid catalyst, so as to produce either a 1,3-dithiane or a 1,2-dithiolane. The reaction is performed in the first flow reactor shown in Figure 1.

Amberlyst-15 (0.055g, 0.231mmol) is inserted into the enclosed area 20 of the tube 10 during manufacture of the first flow reactor described above. Accordingly, the Amberlyst-15 is trapped in the passageway 14 between the first and second plugs 16,18. Amberlyst-15 is a commercially available acid catalyst.

The tube 10 is then mounted in the two supports 22a and 22b as described above. One support 22a was filled with anhydrous acetonitrile and pressure was applied to the open end of the reservoir 24a so as to drive the acetonitrile through the tube 10 and into the reservoir 24b of the other support 22b. In this way, all spaces within the porous plugs 16,18 and between the Amberlyst-15 become filled with acetonitrile.

Excess acetonitrile is then removed from the reservoir 22a and a premixed equimolar solution of a dithiol (1.0M) and either an aldehyde or a ketone (1.0M) in anhydrous acetonitrile, is introduced into the reservoir 22a.

Platinum electrodes are then inserted into the reservoirs 24a,24b and an electric voltage is applied between the platinum electrodes. When the pre-mixed solution contains an aldehyde, the applied voltage is 200 volts per centimetre length of the tube 10. When the premixed solution contains a ketone, the applied voltage is 167 volts per centimetre length of the tube 10.

The applied voltage causes the premixed solution to pass through the first plug/FRIT 16, through the enclosed area 20 containing the Amberlyst-15, through the second plug 18, and into the reservoir 22b. This fluid flow is driven by electroosmotic force.

The reaction products were collected and analysed every ten minutes and the reactions were conducted for a period of 2.5 hour. After the end of the reactions, the reaction products were collected and concentrated in vacuo prior to analysis by NMR spectroscopy.

The results are shown below in Table 1. As shown in Table 1, the conversion and yield for all experiments were greater than 99%. It should also be noted that the flow rates are much greater than are typically achieved in conventional electroosmosis driven flow reactors. This is clearly advantageous as larger amounts of product can be generated more quickly.

**TABLE 1**

| **Starting Material** | **Dithio**l | **Flow Rate (µl min⁻¹)** | **Conversion (%)** | **Actual Yield (g)** | **Yield (%)** |
|---|---|---|---|---|---|
| **Benzaldehyde** | **1,3-Propanedithiol** | **65.0** | **99.92** | **1.960g^{a}** | **99.90** |
| **4-Bromobenzaldehyde** | **1,3-Propanedithiol** | **61.4** | **99.99** | **1.0121** | **99.92** |
| **4-Chlorobenzaldehyde** | **1,3-Propanedithiol** | **61.7** | **99.99** | **0.8561** | **99.91** |
| **4-Cyanobenzaldehyde** | **1,3-Propanedithio**l | **65.4** | **99.99** | **0.8687** | **99.94** |
| **4-Biphenylcarboxaldehyde** | **1,3-Propanedithiol** | **63.0** | **99.99** | **0.8796** | **99.97** |
| **Propiophenone** | **1,3-Propanedithiol** | **40.4** | **100.0** | **0.5467** | **99.94** |
| **Acetophenone** | **1,3-Propanedithiol** | **41.5** | **99.99** | **0.5213** | **99.57** |
| **Cyclohexanone** | **1,3-Propanedithiol** | **42.2** | **99.99** | **0.4742** | **99.62** |
| **Butyrophenone** | **1,3-Propanedithiol** | **41.6** | **99.99** | **0.5935** | **99.90** |
| **4-Nitroacetophenone** | **1,3-Propanedithiol** | **40.9** | **99.99** | **0.6255** | **99.95** |
| **4-Acetylbenzaldehyde** | **1,3-Propanedithiol** | **65.2** | **100.0** | **0.9270** | **99.57** |
| **Benzaldehyde** | **1,2-Ethanedithiol** | **61.5** | **99.99** | **0.6709** | **99.90** |
| **^{a} Reactor operated for 2.5 hours** | | | | | |

Similar results using other ketones are shown in Table 2. In these cases, the dithiol is 1,3-propanedithiol. Because ketones are less reactive than aldehydes, the flow rates need to be less (approximately 40 µl/min) as compared to 60 µm/min for aldehydes.

**TABLE 2**

| **Ketone** | **Flow Rate/µl Min-1** | **Conversion/%** | **Actual Yield/g** | **Yield%** |
|---|---|---|---|---|
| **4-Methylacetophenone** | **42.0** | **99.99 (0.001)** | **0.5947** | **99.91** |
| **2-Methylcyclohexanone** | **41.6** | **99.99 (0.001)** | **0.5040** | **99.96** |
| **Cyclopentanone** | **43.0** | **99.99 (0.001)** | **0.4489** | **99.91** |
| **4-Aminoacetophenone** | **40.3** | **99.99 (0.001)** | **0.5430** | **99.93** |
| **4-Hydroxyacetophenone** | **41.9** | **99.99 (0.0004)** | **0.5680** | **99.90** |

Having demonstrated the ability to protect both aldehydes and ketones, the ability to selectively protect an aldehyde in the presence of a ketone was investigated. By passing a pre-mixed solution of 4-acetylbenzaldehyde and 1-3, propandithiol (1.0M) in anhydrous acetonitrile through the reactor at 65 µl per minute, the aldehyde moiety was selectively protected without reaction of the ketone moiety.

As will be appreciated, other supported acid catalysts may be used instead of the Amberlyst-15. For example, polymer supported p-toluene sulphonic acid may be used. Another suitable catalyst is ytterbium (III) polystyrylsulfonate.

In an adaptation of the previous method, the tube 10 of the first flow reactor is replaced with the tube 32 of the second flow reactor shown in Figure 2 and described above. In this case, the supported acid catalyst is trapped in the first enclosed area 44 during preparation. Copper sulphate impregnated silica gel was inserted into the second enclosed area 46 during preparation. The second flow reactor was used for performing the production of 1,3-dithiane or a 1,2-dithiolane, as described above. However, in this case, the copper sulphate scavenged any unreacted dithiol thereby removing it from solution. This is advantageous as dithiol can poison catalysts used in subsequent reactions.

### Synthesis of α, β - Unsaturated Compounds

The reaction of an aldehyde with an activated methylene in a known flow reactor is described in detail in the article by Wiles et al in Tetrahedron, Volume 60 (2004), 8421-8427. The same reaction was performed in the first flow reactor shown in Figure 1 above.

A premixed solution of aldehyde and activated methylene (1.0M, 1:1) in anhydrous acetonitrile was passed over a packed bed containing silica supported piperazine (0.100 g, 0.170 mmol N) by application of 200 V cm⁻¹. The silica supported piperazine was held in the enclosed area 20 between the porous plugs 16,18 of the first flow reactor. The reaction products were collected every 10 min and analysed by GC-MS and the reaction products combined after 1 hr. The reaction products were concentrated in vacuo and the resulting crude product analysed by NMR spectroscopy. As Table 3 illustrates, in all cases excellent product purities and yields were obtained. In addition, the reaction of benzaldehyde and ethylcyanoacetate was also performed using 3-(dimethylamino)propyl-functionalised silica gel, 3-aminopropyl-functionalised silica gel, 3-(1,3,4,6,7,8-hexahdryo-2H-pyrimido[1,2-1]-pyrimidino)propyl-functionalised silica gel and polymer supported diazabicyclo[2.2.2]octane whereby excellent conversions were obtained (>99.0 %).

**TABLE 3**

| **Aldehyde** | **Activated Methylene** | **Flow Rate (µl min⁻¹)** | **Conversion (%)** | **Actual Yield (g)** | **Yield (%)** |
|---|---|---|---|---|---|
| **Benzaldehyde** | **Ethylcyanoacetate** | **62.0** | **99.98** | **0.87** | **99.70** |
| **4-Bromobenzaldehyde** | **Ethylcyanoacetate** | **55.1** | **99.96** | **2.04^{a}** | **99.35** |
| **Methyl-4-formylBenzoate** | **Ethylcyanoacetate** | **56.1** | **100.0** | **0.87** | **99.80** |
| **3,5-Dimethoxybenzaldehyde** | **Ethylcyanoacetate** | **50.1** | **99.86** | **0.78** | **99.89** |
| **4-Benzyloxybenzaldehyde** | **Ethylcyanoacetate** | **51.1** | **99.99** | **0.90** | **99.67** |
| **Benzaldehyde** | **Malononitrile** | **62.1** | **99.98** | **0.52** | **99.40** |
| **4-Bromobenzaldehyde** | **Malononitrile** | **60.4** | **99.89** | **2.23^{a}** | **99.79** |
| **Methyl-4-formyl Benzoate** | **Malononitrile** | **55.7** | **100.0** | **0.70** | **98.84** |
| **3,5-Dimethoxybenzaldehyde** | **Malononitrile** | **48.4** | **99.87** | **0.64** | **99.17** |
| **4-Benzyloxybenzaldehyde** | **Malononitrile** | **48.3** | **100.0** | **0.75** | **99.73** |
| **^{a} Reactor operated for 2.5 hrs** | | | | | |

### Multi-Step Synthesis of an α, β - Unsaturated Compound

A two-step reaction was then performed in the second flow reactor shown in Figure 2. In this example, the first enclosed area 46 was used to perform an acid catalysed acetal de-protection, so as to produce an aldehyde, as described in an article by Wiles et al in Tetrahedron, Volume 61 (2005) 5209-5217. The second enclosed area 46 was then used for reaction of the aldehyde with an activated methylene as described in the article by Wiles et al in Tetrahedron, Volume 60 (2004), 8421-8427. Using this approach, 100% de-protection of the acetal to aldehyde was observed and 99% conversion of the aldehyde to the desired unsaturated product was observed. An electroosmotic flow rate much greater than those reported in the Wiles article was achieved.

## Claims

1. A method of making a device for electroosmotic flow therein, comprising, preparing a device with a passageway therein and with a porous plug in the passageway, the porous plug being formed by curing a paste comprising filler particles and a silicate solution, the device having an internal surface which borders the passageway and which is contacted by the plug all around the passageway without any gaps therein.

2. A method according to claim 1, wherein the filler particles are glass powder.

3. A method according to claim 2, wherein the filler particles are borosilicate glass powder.

4. A method according to claim 1, wherein the particles have a surface charge when in an electroosmotic liquid.

5. A method according to any one of claims 1 to 4, wherein the particles have a diameter of from 1 µm to 20µm.

6. A method according to claim 5, wherein the particles have a diameter from 2.5 µm to 10 µm.

7. A method according to any preceding claim, wherein the silicate solution is a potassium silicate solution or a sodium silicate solution.

8. A method according to claim 7, wherein the silicate solution is a solution of SiO₂ and K₂O.

9. A method according to any preceding claim, including curing the paste by heating.

10. A method according to any preceding claim, wherein the proportion of the filler particles to the silicate solution is greater than 1:1 by volume.

11. A method according to claim 10, wherein the proportion is about 2:1 by volume.

12. A method according to any preceding claim, wherein the passageway has a maximum cross-sectional dimension of greater than 500µm, or greater than 600 µm, or greater than 700 µm, or greater than 800 µm, or greater than 900µm, or greater than 1000 µm, or greater than 1200µm, or greater than 1400µm, or greater than 1600 µm, or greater than 1800µm, or greater than 2000µm.

13. A method according to any preceding claim, wherein the passageway has a minimum cross-sectional dimension of greater than 500µm, or greater than 600 µm, or greater than 700 µm, or greater than 800 µm, or greater than 900µm, or greater than 1000 µm, or greater than 1200µm, or greater than 1400µm, or greater than 1600 µm, or greater than 1800µm, or greater than 2000µm.

14. A method according to any one of claims 1-13, wherein the passageway has a cross-sectional area of greater than 0.2mm², or greater than 0.3mm², or greater than 0.4mm², or greater than 0.5mm².

15. A method according to any preceding claim, wherein preparing the device comprising providing a tube having a passageway, positioning the paste in the passageway and curing the paste in the passageway.

16. A method according to any one of claims 1 to 14, wherein preparing the device comprises providing a first member with a groove and a second member with a member surface, attaching the members to one another so that the member surface closes the groove to form said passageway.

17. A device made by a method in accordance with any one of claims 1 to 16.

18. A device with a passageway for electroosmotic flow therein, the device having an internal surface which borders the passageway, a porous plug within the passageway, the plug contacting the surface all around the passageway without any gaps therebetween.

19. A device according to claim 18, wherein the porous plug comprises filler particles bound together by silica.

20. A device according to claim 19, wherein the filler particles are glass particles.

21. A device according to claim 20, wherein the filler particles are borosilicate glass particles.

22. A device according to claim 19, wherein the particles have a surface charge when in an electroosmotic environment.

23. A device according to any one of claims 19 to 22, wherein the particles have a diameter of from 1 µm to 20µm.

24. A device according to claim 23, wherein the filler particles have a diameter of from 2.5 µm to 10µm.

25. A device according to any one of claims 18 to 24, wherein the passageway has a maximum cross-sectional dimension of greater than 500µm, or greater than 600 µm, or greater than 700 µm, or greater than 800 µm, or greater than 900µm, or greater than 1000 µm, or greater than 1200µm, or greater than 1400µm, or greater than 1600 µm, or greater than 1800µm, or greater than 2000µm.

26. A device according to any one of claims 18 to 25, wherein the passageway has a minimum cross-sectional dimension of greater than 500µm, or greater than 600 µm, or greater than 700 µm, or greater than 800 µm, or greater than 900µm, or greater than 1000 µm, or greater than 1200µm, or greater than 1400µm, or greater than 1600 µm, or greater than 1800µm, or greater than 2000µm.

27. A device according to any one of claims 18 to 26, wherein the passageway has a cross-sectional area of greater than 0.2mm², or greater than 0.3mm², or greater than 0.4mm², or greater than 0.5mm².

28. A device according to any one of claims 18 to 27, wherein the passageway is formed in a tube.

29. A device according to any one of claims 18 to 27, wherein the device comprises a first member having a groove and a second member having a member surface, the members being attached to one another so that the member surface closes the groove to form the passageway.

30. A device according to any one of claims 17 to 29, wherein the device has a second porous plug in the passageway, a supported catalyst or supported reagent being provided in the passageway and being held between the two porous plugs.

31. A device according to any one of claims 18 to 30, made by a method according to any one of claims 1 to 16.

32. A method of generating electroosmotic flow comprising, providing a device according to any one of claims 17 to 31, filling the passageway and the pores of the or each porous plug with a liquid, and applying an electric voltage across the length of the passageway to cause electroosmotic flow therealong.

33. A method according to claim 32, when dependent on claim 30, wherein the supported catalyst is an acid catalyst and the liquid is a solution comprising a dithiol and either an aldehyde or a ketone.

34. A method according to claim 33, wherein the solution is a solution in acetonitrile.

35. A method according to claim 33 or claim 34, wherein the supported catalyst is Amberlyst-15.

36. A method according to any one of claims 33 to 35, wherein there is a third porous plug in the passageway, the supported catalyst being held between the first mentioned and second plugs and a scavenger being held between the second and third porous plugs, the scavenger being downstream of the supported catalyst in the direction of electroosmotic flow and the scavenger removing any unreacted dithiol from the solution.

37. A method according to claim 36, wherein the scavenger is silica supported copper sulphate.

38. A method according to claim 32, when dependent on claim 30, wherein a supported organic base is held between the first mentioned and second porous plugs, and the solution comprises an aldehyde and an activated methylene.

39. A method according to claim 38, wherein the solution is a solution in acetonitrile.

40. A method according to claim 38 or claim 39, wherein the supported organic base is a primary or secondary amine.

41. A method according to claim 40, wherein the supported organic base is silica supported piperazine.

42. A method according to claim 32, when dependent on claim 30, wherein there is a third porous plug in the passageway, the firstmentioned supported catalyst or reagent being held between the first mentioned and second porous plugs and a second supported catalyst or reagent being held between the second and third porous plugs, the first supported catalyst or reagent being upstream and the second supported catalyst or reagent being downstream in the direction of electroosmotic flow, sequential reactions taking place between the first mentioned and second porous plugs and between the second and third porous plugs.

43. A method according to claim 42, wherein the first mentioned supported catalyst or reagent is an acid catalyst and the second supported catalyst or reagent is an organic base, a solution of a dimethyl acetal and a activated methylene group passing along the passageway with the dimethyl acetal being converted to an aldehyde by the supported acid catalyst and the aldehyde reacting with the activated methylene to form an α,β-unsaturated compound between the second and third porous plugs.

44. A method of preparing a 1,3-dithiane or a 1,2-dithiolane, comprising mixing an aldehyde or a ketone with a dithiol and passing the mixture through a supported acid catalyst so as to produce a 1,3-dithiane or a 1,2-dithiolane.

45. A method according to claim 44, wherein the supported acid catalyst is Amberlyst-15.

46. A method according to claim 44 or 45, wherein the mixture is passed through the supported acid catalyst by electroosmotic force.

47. A method according to claim 46, wherein the mixture is a solution in acetonitrile.

48. A method according to claim 46 or claim 47, wherein the mixture is passed through the device of claim 30; and the supported acid catalyst is held between the first mentioned and second porous plugs.
